# EUROPEAN PATENT APPLICATION

(11) **EP 3 932 486 A1**
(43) Date of publication of application: **05.01.2022**
(21) Application number: 20762621.9
(22) Date of filing: 27.02.2020
(51) Int. Cl.: A61P 7/04, A61P 37/02, A61K 38/17, A61K 38/36, A61K 38/37, A61K 35/407, A61K 35/44

(54) **COMPOSITION FOR TREATING BLOOD COAGULATION AND/OR COMPLEMENT DISORDERS**

(30) Priority: 28.02.2019 JP 2019036542
(71) Applicant: Public University Corporation Yokohama City University, Yokohama-shi, Kanagawa 236-0027 (JP); Takeda Pharmaceutical Company Limited, Osaka-shi, Osaka 541-0045 (JP)
(72) Inventor: TAKEBE Takanori, Tokyo 113-8510 (JP); SAIKI Norikazu, Tokyo 113-8510 (JP); KAWAKAMI Eri, Fujisawa-shi, Kanagawa 251-0012 (JP)
(74) Representative: Jones, Nicholas Andrew
(86) International application number: PCT/JP2020/007886
(87) International publication number: WO 2020/175594

(57) **Abstract**

The present invention provides a composition suitable as a composition for treating blood coagulation and/or complement disorders (raw materials for producing therapeutic agents for these diseases) and a method for effectively producing the composition. A method for producing a composition for treating blood coagulation and/or complement disorders according to the present invention includes the following steps of: (1) embedding an organoid formed from vascular endothelial cells or vascular endothelial cells and hepatocytes, in an extracellular matrix; (2) culturing the extracellular matrix; and (3) collecting a culture supernatant from the culture obtained in the step (2).

SEQUENCE LISTING

<110>  Public University Corporation Yokoham City University
       TAKEDA PHARMACEUTICAL COMPANY LIMITED

<120>  Composition for treatment of coagulation and/or complement
       deficiency disease

<130>  PT38-9044WO

<150>  JP2019-036542
<151>  2019-02-28

<160>  6

<170>  PatentIn version 3.5

<210>  1
<211>  18
<212>  DNA
<213>  Artificial Sequence

<220>
<223>  PCR Forward Primer for HNF4alpha

<400>  1
tcagaccctg agccacct                                                    18


<210>  2
<211>  20
<212>  DNA
<213>  Artificial Sequence

<220>
<223>  PCR Reverse Primer for HNF4alpha

<400>  2
agcaacggac agatgtgtga                                                  20


<210>  3
<211>  23
<212>  DNA
<213>  Artificial Sequence

<220>
<223>  PCR Forward Primer for AFP

<400>  3
tccttgtaag tggcttcttg aac                                              23


<210>  4
<211>  27
<212>  DNA
<213>  Artificial Sequence

<220>
<223>  PCR Reverse Primer for AFP

<400>  4
tgtactgcag agataagttt agctgac                                          27

<210>  5
<211>  20
<212>  DNA
<213>  Artificial Sequence

<220>
<223>  PCR Forward Primer for ALB

<400>  5
cttcccttca tcccgaagtt                                                    20


<210>  6
<211>  19
<212>  DNA
<213>  Artificial Sequence

<220>
<223>  PCR Reverse Primer for ALB

<400>  6
aatgttgcca agctgctga                                                     19

## Description

### Technical Field

The present invention relates to a method for producing a composition for treating blood coagulation and/or complement disorders. More specifically, the present invention relates to a method for producing a composition for treating blood coagulation and/or complement disorders by culturing an organoid. The present invention also relates to a composition for treating blood coagulation and/or complement disorders produced by such a method.

### [Background of the invention]

Hemophilia is a representative disease involved in the blood coagulation system (blood coagulation disorder) and caused by deficiency in a specific blood coagulation factor, more specifically, factor VIII (hemophilia A) or factor IX (hemophilia B). Furthermore, congenital coagulation factor deficiency diseases caused by deficiency and abnormality of other blood coagulation factors (e.g., factor II, factor V, factor VII, factor X and factor XI) are individually classified as hemophilia related diseases. Moreover, deficiency of antithrombin causes thrombosis, whereas deficiency and abnormality of complement components/factors (e.g., complement component 3, complement component 5, Factor B (FB), Factor H (FH)) also cause diseases involved in various complement systems.

Methods used for treating these blood coagulation disorders include administering proteins such as blood coagulation factors. For example, in order to treat hemophilia A, a method is performed involving allowing cultured cells to produce a recombinant protein of human Factor VIII by using an adeno-associated virus (AAV) vector, recovering the recombinant protein, and administering the recombinant protein to patients. However, such a treatment method has problems in that it is difficult to allow cultured cells to express factor VIII by using adeno-associated virus (AAV) vector, and in that an antibody inhibiting the function of a recombinant protein is produced within patient's bodies and decreases a therapeutic effect.

Non-patent literature 1 discloses that when the following organoids (1) to (4) were separately cultured (two-dimensionally) on Matrigel-coated slides, network of vascular endothelial cells finally collapsed in organoids (2) and (3); however, the network survived in the organoids (1) and (4), finally formed fibroblast capsules and separated from the slide upward:
(1) an organoid formed from human microvessel endothelial cells (MVEC) and hepatocytes;
(2) an organoid formed from human umbilical-vein endothelial cells (HUVEC) and hepatocytes;
(3) an organoid formed from rat liver sinusoidal endothelial cells (LSEC) and hepatocytes; and
(4) an organoid formed from human umbilical-vein endothelial cells (HUVEC), normal human dermal fibroblasts (NHDF) and hepatocytes.

Note that, non-patent literature 1 discloses neither that a vascular coagulation factor and/or complements are secreted in the culture supernatant nor that at least the cell supernatant is collected and used as a raw material for producing a composition for treating blood coagulation and/or complement disorders.

### Citation List

### Non Patent Literature

Non patent literature 1: TISSUE ENGINEERING, 12 (6), 2006, pp.1627-1638

### Summary of Invention

### Technical Problem

An object of the present invention is to provide a composition suitable for treating blood coagulation and/or complement disorders (raw materials for producing therapeutic agents for these diseases) and a method for effectively producing the composition.

### Solution to Problem

The present inventors have found that when an organoid formed from vascular endothelial cells or an organoid formed from hepatocytes and vascular endothelial cells were embedded in an extracellular matrix, typically Matrigel (registered trademark), and cultured, a blood coagulation factor and/or complement factors are secreted in the cell supernatant. They have also found that the cell supernatant can be used as a raw material suitable for producing a composition for treating a blood coagulation and/or complement disorders. Based on the findings, the present invention has been accomplished.

More specifically, the present invention provides the followings [1] to [4] in order to attain the aforementioned object.
[1] A method for producing a composition for treating blood coagulation and/or complement disorders, comprising the following steps of:
   (1) embedding an organoid formed from
      vascular endothelial cells or
      vascular endothelial cells and hepatocytes, in an extracellular matrix;
   (2) culturing the extracellular matrix; and
   (3) collecting a culture supernatant from the culture obtained in the step (2).
[2] A culture supernatant obtained by the method according to item 1.
[3] A composition for treating blood coagulation and/or complement disorders, comprising the culture supernatant according to item 2.
[4] A composition for treating blood coagulation and/or complement disorders, comprising two or more components selected from the followings:
   10 × 10⁻⁶ wt% to 1000 × 10⁻⁶ wt% of factor II,
   0.1 × 10⁻⁶ wt% to 10 × 10⁻⁶ wt% of factor V,
   0.1 × 10⁻⁶ wt% to 10 × 10⁻⁶ wt% of factor VII,
   1 × 10⁻⁶ wt% to 100 × 10⁻⁶ wt% of factor VIII,
   0.1 × 10⁻⁶ wt% to 10 × 10⁻⁶ wt% of factor IX,
   0.1 × 10⁻⁶ wt% to 10 × 10⁻⁶ wt% of factor X,
   0.01 × 10⁻⁶ wt% to 1 × 10⁻⁶ wt% of factor XI,
   1 × 10⁻⁶ wt% to 100 × 10⁻⁶ wt% of antithrombin,
   10 × 10⁻⁶ wt% to 1000 × 10⁻⁶ wt% of complement component 3 (C3),
   10 × 10⁻⁶ wt% to 100 × 10⁻⁶ wt% of complement component 5 (C5),
   10 × 10⁻⁶ wt% to 100 × 10⁻⁶ wt% of Factor B (FB), and
   100 × 10⁻⁶ wt% to 1000 × 10⁻⁶ wt% of Factor H (FH) .

### Advantageous Effects of Invention

According to the present invention, a culture supernatant suitable as a composition for treating blood coagulation and/or complement disorders (or raw material for a therapeutic agent for these diseases) can be obtained by embedding an organoid formed from vascular endothelial cells or an organoid formed from vascular endothelial cells and hepatocytes in an extracellular matrix. In the present invention, since the therapeutic composition can be obtained not once but repeatedly from the culture supernatant by continuously culturing the organoid, it is expected that the step of producing the therapeutic composition in a large scale is more efficiently carried out and at lower cost.

### Description of Embodiments

### -Definition-

As used herein, the term "marker" refers to a cell antigen or a gene thereof, such as a "marker protein" and a "marker gene", specifically expressed in a predetermined cell type. Preferably, the marker refers to a cell surface marker. In this case, viable cells can be concentrated, isolated and/or detected. The marker may be a positive selection marker or a negative selection marker.

A marker protein can be detected by use of immunological assay using an antibody specific to a target marker protein and known in the art, for example, ELISA, immunostaining and/or flow cytometry. A marker gene can be detected by a nucleic acid amplification method and/or nucleic acid detection method known in the art, for example, RT-PCR (including quantitative PCR), microarray and/or biochip.

As used herein, "positive" for a marker means that a protein or a gene is expressed at a level (or higher than the level of the background) detectable by a means known in the art.

As used herein, "negative" for a marker means that the expression level of a protein or a gene is less than the detection limit (or lower than the level of the background) by all or any of the means known in the art. The detection limit of expression of a protein or a gene may vary depending on the means.

### -Production method-

The method for producing a composition for treating blood coagulation and/or complement disorders according to the present invention includes the following steps (1) to (3) of:
(1) embedding an organoid formed from
   vascular endothelial cells or
   vascular endothelial cells and hepatocytes, in an extracellular matrix;
(2) culturing the extracellular matrix; and
(3) collecting a culture supernatant from the culture obtained in the step (2).

### • Step (1)

In an embodiment of the present invention, step (1) is step (1A) of embedding an organoid formed from vascular endothelial cells in an extracellular matrix. In an embodiment of the present invention, step (1) is step (1B) of embedding an organoid formed from vascular endothelial cells and hepatocytes in an extracellular matrix.

### • Vascular endothelial cells

The term of vascular endothelial cells in the present invention conceptually includes both hemogenic endothelial cells (HECs) and non-hemogenic endothelial cells (non-HECs). HECs refer to vascular endothelial cells having an ability (hematopoietic ability) to produce hematopoietic stem cells and are also called as blood-cell producing vascular endothelial cells. In the present invention, either HECs or non-HECs may be used and both HECs and non-HECs may be used as the vascular endothelial cells. Alternatively, progenitor cells (described later) of them may be used, or HECs, non-HECs and progenitor cells thereof may be used in any combination.

In an embodiment, if non-HECs alone are used as the vascular endothelial cells, the present invention is carried out by using non-HECs in combination with hepatocytes, more specifically, in accordance with an embodiment of step (1B).

In the specification, the term "hemogenic endothelial cells (HECs)" refer to CD34-positive and CD73-negative vascular endothelial cells having a hematopoietic ability. The HECs to be used in the present invention may include progenitor cells thereof. Examples of the representative progenitor cells include cells present during the differentiation process from Flk-1 (CD309, KDR)-positive progenitor cells (e.g., lateral plate mesoderm cells) of vascular endothelial cells to HECs (see, Cell Reports 2, 553-567, 2012).

The progenitor cells in the early differentiation stage such as Flk-1 (CD309, KDR)-positive cells are the progenitor cells common to HECs and non-HECs. Until otherwise specified, the "HEC progenitor cells" include progenitor cells common to HECs and non-HECs.

In the specification, the term "non-hemogenic endothelial cells (non-HECs)" refer to CD31-, CD73- and CD144-positive vascular endothelial cells having no hematopoietic ability. The non-HECs to be used in the present invention may include progenitor cells thereof. Examples of the representative progenitor cells include cells present during the differentiation process from Flk-1 (CD309, KDR)-positive progenitor cells (e.g., lateral plate mesoderm cells) of vascular endothelial cells to non-HECs (see, Cell Reports 2, 553-567, 2012).

The progenitor cells in the early differentiation stage such as Flk-1 (CD309, KDR)-positive cells are the progenitor cells common to HECs and non-HECs. Until otherwise specified, the "non-HEC progenitor cells" include progenitor cells common to HECs and non-HECs.

The term of vascular endothelial cells to be used in the present invention may be a cell population with high purity of vascular endothelial cells (for example, microvessel endothelial cells (MVEC), liver sinusoidal endothelial cells (LSEC) and umbilical-vein endothelial cells (UVEC)) taken from a living body, or a cell population with high purity of vascular endothelial cells differentiated from pluripotent stem cells, such as ES cells and iPS cells, or other types of cells having an ability to differentiate into vascular endothelial cells. The population with high purity of vascular endothelial cells contains vascular endothelial cells in a ratio of 70% or more, 80% or more, 90% or more, 95% or more, or 99% or more, relative to the total number of cells in the cell population.

The hemogenic endothelial cells (HEC) to be used in the present invention may be a cell population with high purity of HECs taken from a living body or a cell population with high purity of HECs differentiated from pluripotent stem cells, such as ES cells and iPS cells, or other types of cells having an ability to differentiate into vascular endothelial cells (for example, lateral plate mesoderm cells). The cell population with high purity of HECs contains HECs and/or HEC progenitor cells in a ratio of 70% or more, 80% or more, 90% or more, 95% or more, or 99% or more relative to the total number of cells in the cell population.

A method for differentiating the pluripotent stem cells, such as ES cells and iPS cells, or other types of cells having an ability to differentiate into vascular endothelial cells, into hemogenic endothelial cells (HECs) is known in the art. For example, iPS cells can be differentiated in accordance with a method described in, e.g., PLoS One, 2013; 8 (4): e59243, Nat Biotechnol. 2014; 32 (6): 554-61, Sci Rep. 2016; 6: 35680.

In an embodiment, the cell population with high purity of HECs contains HECs in a ratio of 70% or more, 80% or more, 90% or more, 95% or more, or 99% or more relative to the total number of cells in the cell population.

The non-hemogenic endothelial cells (non-HEC) to be used in the present invention may be a cell population with high purity of non-HECs taken from a living body or a cell population with high purity of non-HECs differentiated from pluripotent stem cells, such as ES cells and iPS cells, or other types of cells having an ability to differentiate into vascular endothelial cells (for example, lateral plate mesoderm cells). The cell population with high purity of non-HECs contains non-HECs and/or non-HEC progenitor cells in a ratio of 70% or more, 80% or more, 90% or more, 95% or more, or 99% or more relative to the total number of cells in the cell population.

A method for differentiating the pluripotent stem cells, such as ES cells and iPS cells, or other types of cells having an ability to differentiate into vascular endothelial cells, into non-hemogenic endothelial cells (non-HEC) is known in the art. For example, iPS cells can be differentiated in accordance with a method described in e.g., Nat Cell Biol. 2015; 17 (8): 994-1003 or Cell Rep. 2017; 21 (10): 2661-2670.

In an embodiment, the cell population with high purity of non-HECs contains non-HECs in a ratio of 70% or more, 80% or more, 90% or more, 95% or more, or 99% or more relative to the total number of cells in the cell population.

### • Hepatocytes

The term "hepatocytes" in the present invention, particularly, in step (1B), conceptually includes both hepatocytes which have been differentiated (differentiated hepatocytes) and cells which are committed to differentiation into hepatocytes but have not yet differentiated into hepatocytes (undifferentiated hepatocytes), so-called hepatic progenitor cells (e.g., hepatic endoderm cells). The differentiated hepatocytes may be cells taken from a living body (isolated from the liver of a living body), cells differentiated from pluripotent stem cells, such as ES cells and iPS cells, hepatic progenitor cells or other types of cells obtained differentiating cells having an ability to differentiate into hepatocytes. The undifferentiated hepatocytes may be cells taken from a living body or cells differentiated from pluripotent stem cells, such as ES cells and iPS cells, other stem cells or cells obtained by differentiating progenitor cells. The cells that can be differentiated into hepatocytes can be prepared in accordance with, for example, K. Si-Taiyeb, et al. Hepatology, 51 (1): 297-305 (2010), T. Touboul, et al. Hepatology. 51 (5): 1754-65. (2010). As either of the cell population taken from a living body and the cell population produced by differentiation induction of e.g., ES cells and iPS cells, particularly the latter one, a cell population with high purity of differentiated hepatocytes or undifferentiated hepatocytes may be used; or a cell mixture containing differentiated hepatocytes and undifferentiated hepatocytes in any ratios, may be used.

A method for differentiating pluripotent stem cells, such as ES cells and iPS cells, hepatic progenitor cells, other types of cells having an ability to differentiate into hepatocytes, into hepatocytes is known in the art. For example, iPS cells can be differentiated in accordance with a method described, for example, in Hepatology, 2010; 51 (1): 297-305, Cell Rep. 2017; 21 (10): 2661-2670.

Whether cells are differentiated hepatocytes can be determined by whether the cells are positive for the expression of one or more proteins, such as a mature hepatocyte marker, i.e., asialoglyco protein receptor 1 (ASGR1), an immature hepatocyte marker (early liver differentiation marker), i.e., α-fetoprotein (AFP), an early liver differentiation marker, i.e., albumin (ALB), a retinol-binding protein (RBP4) and transthyretin (TTR), glucose-6-phosphatase (G6PC), or mRNA thereof.

Whether cells are undifferentiated hepatocytes can be determined by whether the cells are positive for the expression of a marker protein, such as HHEX, SOX2, HNF4α, AFP and ALB.

### • Organoid (3D structure)

The organoid to be used in step (1A) is a three-dimensional structure formed from vascular endothelial cells alone. The organoid to be used in step (1B) is a three-dimensional structure formed from vascular endothelial cells and hepatocytes, and is characterized by having a network of vascular channels formed of vascular endothelial cells between hepatocytes. These organoids are generally formed by a three-dimensional culture method known per se (for example, Nature Cell Biology 18, 246-254 (2016)). Alternatively, a three-dimensional culture can be employed in which an extracellular matrix having a single cell embedded therein is cultured to form a three-dimensional structure, thereby producing an organoid, as described later.

The ratio of vascular endothelial cells and hepatocytes for forming the organoid to be used in step (1B) can be controlled to fall within an appropriate range, for example, in consideration that the culture supernatant collected in step (3) contains desired components. In an embodiment of the present invention, the ratio of vascular endothelial cells and hepatocytes (vascular endothelial cells:hepatocytes) is typically 1:0.1 to 5, and preferably 1:0.1 to 2.

In forming an organoid, a culture medium for the organoid is used, which is prepared by mixing a culture medium for vascular endothelial cells and a culture medium (culture solution) for hepatocytes in an appropriate ratio (for example, 1:1).

Examples of the culture medium for vascular endothelial cells include DMEM/F-12 (Gibco), Stempro-34 SFM (Gibco), Essential 6 medium (Gibco), Essential 8 medium (Gibco), EGM (Lonza), BulletKit (Lonza), EGM-2 (Lonza), BulletKit (Lonza), EGM-2 MV (Lonza), VascuLife EnGS Comp Kit (LCT), Human Endothelial-SFM Basal Growth Medium (Invitrogen) and human microvascular endothelial cell growth medium (TOYOBO Co., LTD.).

The culture medium for the vascular endothelial cells may contain one or more additives selected from B27 Supplements (GIBCO), BMP4 (bone formation factor 4), GSK β inhibitor (e.g., CHIR99021), VEGF (vascular endothelial cell growth factor), FGF2 (Fibroblast Growth factor(bFGF (also referred to as basic fibroblast growth factor)), Folskolin, SCF (Stem Cell Factor), TGF β-receptor inhibitor (e.g., SB431542), Flt-3L (Fms-related tyrosine kinase 3 ligand), IL-3 (Interleukin 3), IL-6 (Interleukin 6), TPO (thrombopoietin), hEGF (recombinant human epidermal growth factor), hydrocortisone, ascorbic acid, IGF1, FBS (fetal bovine serum), an antibiotic substance (for example, gentamicin, amphotericin B), heparin, L-glutamine, phenol red, BBE, and so on. The amounts of these additives to be added can be appropriately determined by those skilled in the art with reference to ordinary culture conditions for vascular endothelial cells.

Examples of the culture medium for hepatocytes include RPMI (FUJIFILM Corporation) and HCM (Lonza).

The culture medium for hepatocytes may contain one or more additives selected from Wnt3a, activin A, BMP4, FGF2, FBS (fetal bovine serum), HGF (hepatocyte growth factor H), oncostatin M (OSM), dexamethasone (Dex), and so on. The amounts of these additives to be added can be appropriately determined by those skilled in the art with reference to ordinary culture conditions for hepatocytes.

Alternatively, examples of the culture medium for hepatocytes include a medium for hepatocytes containing at least one selected from ascorbic acid, BSA-FAF, insulin, hydrocortisone and GA-1000; an HCM BulletKit (Lonza) without hEGF (recombinant human epidermal growth factor); an RPMI1640 (Sigma-Aldrich) supplemented with 1% B27 Supplements (GIBCO) and 10 ng/mL hHGF (Sigma-Aldrich); and a medium prepared by mixing GM BulletKit (Lonza) and an HCM BulletKit (Lonza) without hEGF (recombinant human epidermal growth factor) in a ratio of 1:1 and further supplemented with dexamethasone, oncostatin M and HGF.

In forming an organoid, culture conditions are ordinarily 5% CO₂ and a culture temperature of 30 to 40°C, preferably about 37°C. The culture period is ordinarily 1 to 2 days.

A culture vessel for use in formation of an organoid can be appropriately selected depending on the size of the organoid to be formed. As such a culture vessel, a culture vessel having an equivalent diameter of 20 µm or more and 2.5 mm or less and a depth of 20 µm or more and 1000 µm or less, as described in, e.g., WO2014/199622, and a commercially available culture vessel (Elplasia (Kuraray Co., LTD.)) can be preferable used. Furthermore, as the culture vessel, a vessel made of a material that prevents an organoid from adhering to the surface or a surface-treated vessel is preferred.

Whether the organoid to be used in step (1B) is formed can be determined based on one or more of the following:
(i) shape (e.g., presence of, 3D structure),
(ii) presence of albumin in culture supernatant,
(iii) be positive for hepatocyte marker (e.g., AFP, ALB, HNF4α).

It should be noted that "mesenchymal cells" are not used for forming an organoid in the present invention. The term "mesenchymal cells" refers to connective-tissue cells, which are principally present in the connective tissue derived from the mesoderm and form a support structure of cells functioning in the tissue, and conceptually includes both differentiated cells (differentiated mesenchymal cells) and cells which are committed to differentiation into mesenchymal cells but have not yet differentiated into mesenchymal cells (undifferentiated mesenchymal cells), so-called mesenchymal stem cells. The cells represented by the terms used by those skilled in the art, such as mesenchymal stem cells, mesenchymal progenitor cells and mesenchymal cells (R. Peters, et al. PLoS One. 30;5 (12): e15689. (2010)), are equivalent to the "mesenchymal cells" in the specification. Note that the term "vascular endothelial cells", although they are cells differentiated from undifferentiated mesenchymal cells, are out of definition of "mesenchymal cells" in the specification. More specifically, in the present invention, neither the term "undifferentiated mesenchymal cells" nor the term "differentiated mesenchymal cells" except vascular endothelial cells are used in a step of forming an organoid.

Whether cells are undifferentiated mesenchymal cells or differentiated mesenchymal cells can be determined by whether the cells are positive for the expression of one or more proteins of, for example, Stro-1, CD29, CD44, CD73, CD90, CD105, CD133, CD271 and Nestin, which serve as undifferentiated mesenchymal cell markers, or mRNA thereof (if positive, the cells are determined as undifferentiated mesenchymal cells; and if negative, the cells are determined as differentiated mesenchymal cells).

### • Extracellular matrix

As the extracellular matrix in the present invention, an extracellular matrix ordinarily used, that is, solid at room temperature or more and used for cell culture, particularly 3D cell culture, can be employed. The extracellular matrix contains a fibrous protein and proteoglycan as major components. Examples of such components include, elastin, entactin, osteonectin, collagen, tenascin, thromboxane, perlecan, vitronectin, fibrillin, fibronectin, heparin (sulfate) and laminin. A basal membrane matrix known as a product name "Matrigel" (Corning) and containing laminin, collagen IV and entactin together with a growth factor such as EGF, IGF-1, PDGF and TGF-β, is a preferable example of the extracellular matrix in the present invention. Also, a self-assembly peptide known as a hydrogel, for example, a hydrogel containing arginine, glycine and asparagine, can be used as the extracellular matrix in the present invention. The composition and concentration (undiluted solution or diluted solution) of the extracellular matrix can be appropriately controlled by those skilled in the art such that the extracellular matrix in which the organoid to be obtained is embedded (simply referred to as an "organoid embedded matrix"), has an appropriate hardness.

The extracellular matrix in which an organoid formed from vascular endothelial cells are embedded, or the extracellular matrix in which an organoid formed from vascular endothelial cells and hepatocytes are embedded, can be formed by adding an appropriate amount of extracellular matrix containing appropriate components in, e.g., wells of a culture vessel containing an organoid (if necessary, an ionic solution or an ionic molecule for solidifying hydrogel is further added).

An organoid embedded matrix is preferably prepared such that an organoid formed from vascular endothelial cells in step (1A) or an organoid formed from vascular endothelial cells and hepatocytes in step (1B), are held in an extracellular matrix having an appropriate hardness. For example, if an extracellular matrix (e.g., Matrigel) is added in a relatively large amount of a culture medium having the organoid suspended therein, the hardness of the extracellular matrix becomes not sufficient, with the result that the organoid precipitates and easily attaches to the culture vessel. Because of this, the collection rate of the culture supernatant containing desired components may decrease in steps (2) and (3).

Note that, the organoid embedded matrix in the present invention can be obtained typically in accordance with so-called a 3D cell culture method, more specifically, obtained in accordance with the aforementioned procedure, which includes mixing an organoid formed from vascular endothelial cells (or a single vascular endothelial cell if an organoid is formed within an extracellular matrix), or an organoid formed from vascular endothelial cells and hepatocytes, with components of an extracellular matrix, followed by solidifying it. Accordingly, a structure, which is obtained in accordance with a two-dimensional coating method, more specifically, by forming a coating layer of an extracellular matrix previously solidified to a predetermined degree in a culture vessel such as a plate well, and seeding an organoid (or cells constituting a single organoid), with the result that an organoid grows on the coating layer or an organoid migrates into the coating layer with time and grow in a suspended or precipitated state, does not correspond to the organoid embedded matrix of the present invention.

In contrast, in an embodiment of step (1), the following (i), (ii) and (iii) all correspond to the organoid embedded matrix of the present invention:
(i) an organoid embedded matrix obtained by forming an organoid from vascular endothelial cells alone or vascular endothelial cells and hepatocytes, then isolating the organoid, and mixing them with an extracellular matrix,
(ii) an organoid embedded matrix obtained by forming an organoid from vascular endothelial cells alone or vascular endothelial cells and hepatocytes by 3D culture and then mixing the organoid with a matrix, and
(iii) an organoid embedded matrix obtained by adding an extracellular matrix to vascular endothelial cells alone or a cell mixture of vascular endothelial cells and hepatocytes in advance in accordance with 3D cell culture method to form a cell mixture embedded matrix and culturing the embedded cell mixture to form an organoid (3D structure) (the organoid embedded matrix can be used as it is for culture in subsequent step (2)).

A method for embedding an organoid or cells in an extracellular matrix can be carried out representatively by mixing a liquid-state extracellular matrix (e.g., Matrigel) at 4°C or less and an organoid or cells with stirring and allowing the mixture to stand still at 37°C or more to solidify the extracellular matrix.

### • Step (2)

Step (2) is a step of culturing the extracellular matrix (organoid embedded matrix) in which an organoid formed from vascular endothelial cells or formed from vascular endothelial cells and hepatocytes prepared in step (1), are embedded.

Culturing the organoid embedded matrix in step (2) may be carried out in appropriate conditions such that a culture supernatant containing desired components at desired concentrations can be collected in the following step (3). For example, in step (2), the culture temperature is ordinarily 30 to 40°C preferably about 37°C at 5% CO₂ and the culture period is usually 10 to 50 days.

In the present invention, culturing the organoid embedded matrix in step (2) can be carried out typically by suspension culture.

The shape, material, size (culture scale) and others of the culture vessel are not particularly limited. If necessary, the culture vessel may be equipped with a means for enabling culturing with stirring or shaking. The culture vessel is preferably formed of a material that prevents adhesion of an organoid embedded matrix to the surface or a surface-treated vessel. For example, a commercially available low-adsorption culture vessel (Corning) can be used.

As the culture medium for the organoid embedded extracellular matrix in step (2), a conditioned medium is preferably used in consideration that secretion of a blood coagulation factor or complements (components (a) to (1) that will be described in the following step (3)) from vascular endothelial cells or an organoid is promoted and a culture supernatant containing individual components at higher concentrations can be obtained in step (3); or that a culture supernatant containing individual components can be obtained at desired concentrations in a shorter period, or in consideration of the ratio of vascular endothelial cells and hepatocytes used in formation of an organoid.

In an embodiment of the present invention, as the culture medium for an organoid embedded extracellular matrix, a culture medium for the vascular endothelial cells is used.

In another embodiment of the present invention, as the culture medium for organoid embedded extracellular matrix, a culture medium for organoid, which is prepared by mixing a medium for vascular endothelial cells and a medium for hepatocytes, can be used. The mixing-ratio of these mediums can be appropriately controlled by those skilled in the art so as to obtain desired components (blood coagulation factor and/or complements).

In an embodiment of the present invention, the culture medium in step (2) is a medium containing serum having a concentration within an appropriate range, preferably a concentration of from 0% (serum-free culture medium) to 5%. Note that, as the serum, a "serum substitute" can be used. In this case, the concentration range of the serum substitute can be set so as to fall within the equivalent range of the serum.

In an embodiment of the present invention, the culture medium in step (2) is a medium supplemented with a Nrf2 (nuclear factor erythroid-2-related factor 2) activator. Examples of the Nrf2 activator include R-α-lipoic acid, tert-butyl hydroquinone, sulforaphane, a *Polygonum cuspidatum* extract containing resveratrol and BM (bardoxolone methyl)). The amount of these additives can be appropriately determined by those skilled in the art with reference to ordinary culture conditions for culturing vascular endothelial cells and hepatocytes. In the embodiment, the concentration of the Nrf2 activator in the culture medium is preferably 20 nM or more.

### • Step (3)

Step (3) is a step for collecting the culture supernatant from a culture obtained after culturing in step (2). Owing to step (3), it is possible to collect a culture supernatant containing components such as a blood coagulation factor secreted from vascular endothelial cells and/or hepatocytes and complements at preferable concentrations for producing a composition for treating blood coagulation and/or complement disorders.

In an embodiment of the present invention, the culture supernatant collected in step (3) contains two or more components (blood coagulation factor and/or complements) selected from the following (a) to (1), preferably factor VIII and one or more components except factor VIII.
(a) 10 × 10⁻⁶ wt% to 1000 × 10⁻⁶ wt% of factor II,
(b) 0.1 × 10⁻⁶ wt% to 10 × 10⁻⁶ wt% of factor V,
(c) 0.1 × 10⁻⁶ wt% to 10 × 10⁻⁶ wt% of factor VII,
(d) 1 × 10⁻⁶ wt% to 100 × 10⁻⁶ wt% of factor VIII,
(e) 0.1 × 10⁻⁶ wt% to 10 × 10⁻⁶ wt% of factor IX,
(f) 0.1 × 10⁻⁶ wt% to 10 × 10⁻⁶ wt% of factor X,
(g) 0.01 × 10⁻⁶ wt% to 1 × 10⁻⁶ wt% of factor XI,
(h) 1 × 10⁻⁶ wt% to 100 × 10⁻⁶ wt% of antithrombin,
(i) 10 × 10⁻⁶ wt% to 1000 × 10⁻⁶ wt% of complement component 3 (C3),
(j) 10 × 10⁻⁶ wt% to 100 × 10⁻⁶ wt% of complement component 5 (C5),
(k) 10 × 10⁻⁶ wt% to 100 × 10⁻⁶ wt% of Factor B (FB), and
(l) 100 × 10⁻⁶ wt% to 1000 × 10⁻⁶ wt% of Factor H (FH) .

Detection and quantification of a blood coagulation factor and complements can be carried out by a method known per se, for example, ELISA (enzyme-linked immunosorbent assay), and a kit for ELISA is commercially available [e.g., Human Coagulation Factor II (F2) ELISA Kit (Biomatik), Human Coagulation Factor V (F5) ELISA Kit (Biomatik), Human Coagulation Factor VII (F7) ELISA Kit (Biomatik), Human Coagulation Factor VIII (F8) ELISA Kit (Biomatik), Human Coagulation Factor IX (F9) ELISA Kit (Biomatik), Human Coagulation Factor X (F10) ELISA Kit (Biomatik), Human Coagulation Factor XI (F11) ELISA Kit (Biomatik), High Sensitive Human Albumin (ALB) ELISA Kit (Biomatik), (C3): Complement C3 Human ELISA kit (Abcam), (C5): Complement C5 Human ELISA kit (Abcam), Human Factor H ELISA Kit (Abcam), Human Factor B ELISA Kit (Abcam)].

The culture supernatant collected in step (3) may be concentrated if necessary. The concentrated culture supernatant becomes favorable for use in a composition for treating blood coagulation and/or complement disorders, since, e.g., the activity based on activated partial thromboplastin time (APTT) increases (APTT is reduced).

Specific examples of the blood coagulation disorder include hemophilia (hemophilia A (factor VIII deficiency), hemophilia B (factor IX deficiency)), hemophilia-related diseases (factor II deficiency, factor V deficiency, factor VII deficiency, factor X deficiency, factor XI deficiency), and thrombosis (antithrombin deficiency). Specific examples of the complement disorder include complement component 3 deficiency, complement component 5 deficiency, Factor B deficiency and Factor H deficiency.

Since the culture supernatant collected in step (3) and the concentrated culture supernatant are low in toxicity (e.g., acute toxicity, chronic toxicity, genotoxicity, reproductive toxicity, cardiotoxicity, carcinogenic), the culture supernatants can be directly used or used together with pharmaceutically acceptable additives, as pharmaceutical products safe in administration (typically, parenterally) to mammals (for example, mouse, rat, hamster, rabbit, cat, dog, cow, sheep, monkey, human).

Alternatively, any blood coagulation factor and/or complements in the culture supernatant may be separated by a method known per se, and concentrated as needed, and directly or blended with pharmaceutically acceptable additives used in pharmaceutical products to prepare a medical product.

The contents of the blood coagulation factor and/or complement in the medical product are appropriately determined depending on, e.g., the administration target (sex, age, body weight), administration route, disease and symptomatology.

Now, the present invention will be more specifically described by way of Examples but the present invention is not limited by them.

### Examples

In Examples, confirmation of cell markers was carried out by flow cytometry, immunostaining and/or quantitative PCR.

### (Confirmation of CD31, CD34, CD73 and CD144 markers)

Confirmation of CD31, CD34, CD73 and CD144 markers was carried out by reacting cells with fluorescent labeled anti-CD31 antibody (FITC Mouse anti-Human CD31, BD Pharmingen), anti-CD34 antibody (APC anti-human CD34 Antibody, BioLegend), anti-CD73 antibody (CD73-PE, human, Miltenyi Biotec or CD73-APC, human, Miltenyi Biotec) and anti-CD144 antibody (PE Mouse anti-Human CD144, BD Pharmingen), and thereafter subjecting the cells to flow cytometry (FACS Fortessa (BD)). As a marker negative sample, undifferentiated human iPS cells (1383D2; Center for iPS Cell Research and Application, Kyoto University) were used. Also, negative controls were prepared by reacting antibodies, which are the same isotypes as the labeled antibodies and do not recognize target markers.

### (Confirmation of HNF4α marker)

Confirmation of HNF4α marker was carried out by immunostaining and quantitative PCR.

### (Immunostaining method)

Cells were fixed with 4 % paraformaldehyde (PFA) in PBS at room temperature for 15 minutes. Blocking was carried out with donkey and goat sera. The cells were immune-stained with an anti-HNF4α antibody (Santa-Cruz) and a fluorescently labeled secondary antibody (Novex Donkey anti-Goat IgG (H+L) Secondary Antibody (Invitrogen)) binding to the anti-HNF4α antibody, and then, observed by a fluorescence microscope.

### (Quantitative PCR)

RNA was extracted from cells by use of PureLink RNA Mini Kit (Invitrogen) and cDNA was synthesized by use of High-Capacity cDNA Reverse Transcription Kit (Invitrogen). Quantitative PCR was carried out by use of THUNDERBIRD Probe qPCR Mix (TOYOBO Co., LTD.) and QuantStudio 7Flex real time PCR system (Applied Biosystems). The PCR primers (Fw: forward primer; Rv: reverse primer) and probe used herein are as follows:
HNF4 α
   Fw 5'-TCAGACCCTGAGCCACCT-3' (SEQ ID No. 1)
   Rv 5'-AGCAACGGACAGATGTGTGA-3' (SEQ ID No. 2)
probe; Universal ProbeLibrary Probe 027 (Roche)
AFP
   Fw 5'-TCCTTGTAAGTGGCTTCTTGAAC-3' (SEQ ID No. 3)
   Rv 5'-TGTACTGCAGAGATAAGTTTAGCTGAC-3' (SEQ ID No. 4)
probe; Universal ProbeLibrary Probe 061 (Roche)
ALB
   Fw 5'-CTTCCCTTCATCCCGAAGTT-3' (SEQ ID No. 5)
   Rv 5'-AATGTTGCCAAGCTGCTGA-3' (SEQ ID No. 6)
probe; Universal ProbeLibrary Probe 027 (Roche)
18s rRNA (Endogenous Control)
EUK 18s rRNA (20×) (ABI)

### [Example 1] Preparation of organoids using non-hemogenic endothelial cells and hepatocytes and production of blood coagulation factor and complement factor from the organoids

### (1-1) Preparation of human non-hemogenic endothelial cells (non-HEC)

Human iPS cells (1383D2; Center for iPS Cell Research and Application, Kyoto University) were added in DMEM/F-12 (Gibco) (10 ml) supplemented with 1% B-27 Supplements (GIBCO), BMP4 (25 ng/ml) and CHIR99021 (8 µM). Culture was carried out in the conditions: 5% CO₂ and 37°C, for 3 days to induce mesodermal cells. To Stempro-34 SFM (Gibco) (10 ml), VEGF (200 ng/ml) and Folskolin (2 µM) were supplemented. Culture was carried out in the conditions: 5% CO₂ and 37°C, for 7 days to obtain a CD31-positive, CD73-positive and CD144-positive human non-hemogenic endothelial cell population.

### (1-2) Preparation of human hepatic endoderm cells (HE)

Human iPS cells (1383D2; Center for iPS Cell Research and Application, Kyoto University) were added in RPMI (FUJIFILM Corporation) (2 ml) supplemented with Wnt3a (50 ng/ml) and activin A (100 ng/ml). Culture was carried out in the conditions: 5% CO₂ and 37°C for 5 days to induce endoderm cells. The endoderm cells obtained were cultured in the same medium supplemented with 1% B27 Supplements (GIBCO) and FGF2 (10 ng/ml). Culture was carried out in the conditions: 5% CO₂ and 37°C for further 5 days to obtain an AFP, ALB and HNF4α-positive human hepatic endoderm cell population.

### (1-3) Preparation of organoids (3D structure)

The human hepatic endoderm cells (HE) and human non-hemogenic endothelial cells (non-HEC) prepared were mixed in a ratio of 10:7 (total cell number: 18 × 10⁵) and co-cultured in a 3D culture vessel, Elplasia (Kuraray) for a day in the conditions: 5% CO₂ and 37°C, to prepare aggregates. The medium used in the co-culture was prepared by mixing a medium (A) for hepatocytes, which was an HCM (Lonza) supplemented with FBS (5%), HGF (10 ng/ml), OSM (20 ng/ml) and Dex (100 nM), and a medium (A) for vascular endothelial cells, which was a Stempro-34 SFM (Gibco) supplemented with VEGF (50 ng/ml) and FGF2 (10 ng/ml), in a volume ratio of 1:1 (referred to as "a medium (A) for organoids" in the specification), and used in a volume of 2 ml.

### (1-4) Embedding in extracellular matrix

The organoids (3D structure), which were aggregates of human hepatic endoderm cells (HE) and human non-hemogenic endothelial cells (non-HEC) obtained in accordance with the procedure of the above section (1-3), were admixed with Matrigel (BD pharmingen) at 4°C, and then, raised in temperature up to 37°C. In this manner, the organoids were homogenously embedded in Matrigel. The organoids embedded in Matrigel were suspension-cultured in a low-adsorption culture dish (Corning) in the conditions: 5% CO₂ and 37°C. As the culture medium, the same medium (A) for organoids as in the above section (1-3) was used in a volume of 4 ml. From Day 21 to about Day 60 after initiation of the suspension culture, the culture solution (culture supernatant) was taken with time and the yields of a blood coagulation factor (factor VIII) and complement factors (complement component 3 (C3), complement component 5 (C5), Factor H (FH) and Factor B (FB)) were measured.

The yields of factor VIII, complement component 3 (C3), complement component 5 (C5), Factor H (FH) and Factor B (FB) were measured by using the following kits according to ELISA (enzyme-linked immunosorbent assay), respectively.
Factor VIII: Human Coagulation Factor VIII (F8) ELISA Kit (Biomatik),
Complement component 3 (C3): Complement C3 Human ELISA kit (Abcam),
Complement component 5 (C5): Complement C5 Human ELISA kit (Abcam),
Factor H (FH): Human Factor H ELISA Kit (Abcam),
Factor B (FB): Human Factor B ELISA Kit (Abcam).

### (1-5) Change of culture medium

The yields of the blood coagulation factor (factor VIII) and complement factors (complement component 3 (C3), complement component 5 (C5), Factor H (FH) and Factor B (FB)) were measured in the same procedure as in the above sections (1-3) and (1-4) except that the medium (A) for hepatocytes alone or the medium (A) for vascular endothelial cells alone was used in place of the medium (A) for organoids.

### (1-6) Change of cell mixing-ratio

The yields of the blood coagulation factor (factor VIII) and complement factors (complement component 3 (C3), complement component 5 (C5), Factor H (FH) and Factor B (FB)) were measured in the same procedure as in the above sections (1-3) and (1-4) except that the organoids obtained from a cell population having a ratio of HEs:non-HECs = 1:1 (7:7), 4:7, 1:7 or 0:7 (total cell number of each population: 18 × 10⁵) were used in place of the organoids obtained from the cell population having human hepatic endoderm cells (HE) and human non-hemogenic endothelial cells (non-HEC) in a ratio of 10:7 (total cell number: 18 × 10⁵) and by changing the culture solution as described in the above section (1-5).

### (Results)

The measurement results of the concentration (pg/million cells) of factor VIII contained in the culture solution (culture supernatant) on Day 60 after initiation of culture are shown in the following table. Factor VIII was produced from the organoids embedded in Matrigel and cultured in accordance with the present invention. In particular, in the case where a medium (A) for organoids was used as the culture medium, the yield of factor VIII was high.

**[Table 1]**

| Cells used for preparation of organoids | Culture condition | | Yield of Factor VIII (pg/million cells; Mean±S.D.) |
|---|---|---|---|
| | | | Factor VIII |
| HE:non-HEC =10:7 | Embedded in Matrigel | Medium (A) for vascular endothelial cells | 5343.1 ± 3767.7 |
| | | Medium (A) for organoids | 63955.5 ± 4709.7 |
| HE:non-HEC =1:1 | Embedded in Matrigel | Medium (A) for vascular endothelial cells | 9472.6 ± 6216.8 |
| | | Medium (A) for organoids | 59493.0 ± 9890.3 |
| HE:non-HEC =4:7 | Embedded in Matrigel | Medium (A) for vascular endothelial cells | 5742.7 ± 1883.9 |
| | | Medium (A) for organoids | 47570.7 ± 16766.5 |
| HE:non-HEC =1:7 | Embedded in Matrigel | Medium (A) for vascular endothelial cells | 5742.7 ± 565.2 |
| | | Medium (A) for organoids | 32184.9 ± 10267.1 |
| HE:non-HEC =0:7 | Embedded in Matrigel | Medium (A) for vascular endothelial cells | 6075.8 ± 5369.0 |
| | | Medium (A) for organoids | 51966.6 ± 31272.3 |

The measurement results of concentrations (ng/million cells) of complement factors (complement component 3 (C3), complement component 5 (C5), Factor B (FB) and Factor H (FH)) contained in the culture solution (culture supernatant) on Day 30 after initiation of culture are shown in the following table. Individual complement factors were produced from the organoid formed from non-hemogenic endothelial cells and hepatocytes embedded in Matrigel and cultured in accordance with the present invention. In particular, in the case where the medium (A) for organoids was used as the culture medium, the yields of the complement factors were high.

**[Table 2]**

| Cells used for preparation of organoids | Culture condition | | Yield of complement (ng/million cells; Mean±S.D.) | | | |
|---|---|---|---|---|---|---|
| | | | C3 | C5 | FH | FB |
| HE:non-HEC =10:7 | Embedded in Matrigel | Medium (A) for vascular endothelial cells | 502.0 ± 64.1 | 34.2 ± 1.6 | n.d. | n.d. |
| | | Medium (A) for organoids | 2670.5 ± 4.1 | 358.4 ± 5.5 | 1083.4 ± 61.3 | 3401.7 ± 0.0 |
| HE:non-HEC =1:1 | Embedded in Matrigel | Medium (A) for vascular endothelial cells | 604.4 ± 16.4 | 39.2 ± 4.2 | n.d. | n.d. |
| | | Medium (A) for organoids | 2611.7 ± 46.1 | 309.3 ± 52.4 | 905.3 ± 50.4 | 3008.1 ± 62.5 |
| HE:non-HEC =4:7 | Embedded in Matrigel | Medium (A) for vascular endothelial cells | 668.9 ± 78.1 | 39.5 ± 9.0 | n.d. | n.d. |
| | | Medium (A) for organoids | 2461.7 ± 187.5 | 323.7 ± 68.8 | 912.2 ± 34.3 | 3198.3 ± 562.9 |
| HE:non-HEC =1:7 | Embedded in Matrigel | Medium (A) for vascular endothelial cells | 661.4 ± 121.7 | 22.4 ± 2.3 | n.d. | n.d. |
| | | Medium (A) for organoids | 2543.7 ± 81.4 | 251.1 ± 10.0 | 838.1 ± 41.6 | 2490.7 ± 6.3 |
| HE:non-HEC =0:7 | Embedded in Matrigel | Medium (A) for vascular endothelial cells | n.d. | n.d. | n.d. | n.d. |
| | | Medium (A) for organoids | n.d. | n.d. | 60.6 ±6.8 | n.d. |

| | | | | | | |
|---|---|---|---|---|---|---|
| [n.d.] Not detected | | | | | | |

### [Example 2] Preparation of hemogenic endothelial cells (HEC) and production of blood coagulation factor and complement factor from HEC

### (Experimental method)

### (2-1) Preparation of human hemogenic endothelial cells (HEC)

Human iPS cells (1383D2; Center for iPS Cell Research and Application, Kyoto University) were cultured in the presence of AK02N (Ajinomoto) (8 ml) in the conditions: 5% CO₂ and 37°C, for 6 to 7 days to form iPS-cell colonies having a diameter of 500 to 700 µm. Then, Essential 8 medium (Gibco) (8 ml) were supplemented with BMP4 (80 ng/ml), VEGF (80 ng/ml) and CHIR99021 (2 µM), and culture was carried out in the conditions: 5% CO₂ and 37°C, for 2 days. Next, the medium was exchanged with an Essential 6 medium (Gibco) (8 ml) supplemented with VEGF (80 ng/ml), FGF2 (25 ng/ml), SCF (50 ng/ml) and SB431542 (2 µM). Culture was carried out in the conditions: 5% CO₂ and 37°C, for further 2 days to induce lateral plate mesoderm cells. Thereafter, the medium was exchanged with a Stempro-34 SFM (Gibco) (8 ml) supplemented with VEGF (80 ng/ml), SCF (50 ng/ml), Flt-3L (50 ng/ml), IL-3 (50 ng/ml), IL-6 (50 ng/ml) and TPO (5 ng/ml). Culture was carried out in the conditions: 5% CO₂ and 37°C, for 2 days. The medium was further exchanged with a culture medium prepared by excluding VEGF from the composition of the above culture medium. Culture was carried out in the conditions: 5% CO₂ and 37°C for a day to obtain CD34-positive and CD73-negative hemogenic endothelial cells.

### (2-2) Embedding of HECs in extracellular matrix and formation of organoids

The human hemogenic endothelial cells (HEC) prepared in the above section (2-1) were admixed wtih Matrigel (BD pharmingen) at 4°C, and then, raised in temperature up to 37°C. In this manner, the cells were homogenously embedded in Matrigel to prepare HEC aggregates (organoids). Then, the organoids embedded in Matrigel were cultured in a low-adsorption culture dish (Corning) in the conditions: 5% CO₂ and 37°C. As the culture medium, the medium (A) for organoids was used. From Day 21 to about Day 42 after initiation of the culture, the culture solution was taken with time and the yields of the blood coagulation factor (factor VIII) and complement factors (complement component 3 (C3), complement component 5 (C5), Factor H (FH) and Factor B (FB)) were measured in the same manner as in the above section (1-4).

### (Results)

The measurement results of the concentration (pg/million cells) of factor VIII contained in the culture solution on Day 21 after initiation of culture are shown in the following table. Factor VIII was produced in a large yield from the hemogenic endothelial cells (HEC) embedded in Matrigel and cultured in accordance with the present invention.

**[Table 3]**

| Cells used for preparation of organoids | Culture condition | Yield of Factor VIII (pg/million cells; Mean±S.E.) |
|---|---|---|
| | | Factor VIII |
| HEC | Embedded in Matrigel Medium (A) for organoids | 870,363.8 ± 58,272.9 |

The measurement results of concentrations (ng/million cells) of complement factors (complement component 3 (C3), complement component 5 (C5), Factor B (FB) and Factor H (FH)) contained in the culture solution on Day 21 after initiation of culture are shown in the following table. Individual complement factors were produced from the organoid formed from hemogenic endothelial cells (HEC) and embedded in Matrigel and cultured in accordance with the present invention. In particular, the yields of complement component 3 (C3) and Factor H (FH) were high.

**[Table 4]**

| Cells used for preparation of organoids | Culture condition | Yield of complement (ng/million cells; Mean±S.E.) | | | |
|---|---|---|---|---|---|
| | | C3 | C5 | FH | FB |
| HEC | Embedded in Matrigel Medium (A) for organoids | 1,111.7 ± 93.8 | 129.4 ± 9.4 | 705.6 ± 101.1 | 229.0 ± 29.3 |

## Claims

1. A method for producing a composition for treating blood coagulation and/or complement disorders, comprising the following steps of:
(1) embedding an organoid formed from
vascular endothelial cells or
vascular endothelial cells and hepatocytes, in an extracellular matrix;
(2) culturing the extracellular matrix; and
(3) collecting a culture supernatant from the culture obtained in step (2).

2. A culture supernatant obtained by the method according to claim 1.

3. A composition for treating blood coagulation and/or complement disorders, comprising the culture supernatant according to claim 2.

4. A composition for treating blood coagulation and/or complement disorders, comprising two or more components selected from:
10 × 10⁻⁶ wt% to 1000 × 10⁻⁶ wt% of factor II,
0.1 × 10⁻⁶ wt% to 10 × 10⁻⁶ wt% of factor V,
0.1 × 10⁻⁶ wt% to 10 × 10⁻⁶ wt% of factor VII,
1 × 10⁻⁶ wt% to 100 × 10⁻⁶ wt% of factor VIII,
0.1 × 10⁻⁶ wt% to 10 × 10⁻⁶ wt% of factor IX,
0.1 × 10⁻⁶ wt% to 10 × 10⁻⁶ wt% of factor X,
0.01 × 10⁻⁶ wt% to 1 × 10⁻⁶ wt% of factor XI,
1 × 10⁻⁶ wt% to 100 × 10⁻⁶ wt% of antithrombin,
10 × 10⁻⁶ wt% to 1000 × 10⁻⁶ wt% of complement component 3 (C3),
10 × 10⁻⁶ wt% to 100 × 10⁻⁶ wt% of complement component 5 (C5),
10 × 10⁻⁶ wt% to 100 × 10⁻⁶ wt% of Factor B (FB), and
100 × 10⁻⁶ wt% to 1000 × 10⁻⁶ wt% of Factor H (FH) .
